# EUROPÄISCHE PATENTSCHRIFT

(11) **EP 1 200 544 B1**
(45) Veröffentlichungstag und Bekanntmachung des Hinweises auf die Patenterteilung: **09.11.2005**
(21) Anmeldenummer: 00953082.5
(22) Anmeldetag: 22.07.2000
(51) Int. Cl.: C11D 3/37, C11D 3/33, C11D 3/20

(54) **ENTFERNUNG PIGMENTHALTIGER RÜCKSTÄNDE IN DER PHARMAZEUTISCHEN ODER KOSMETISCHEN INDUSTRIE**
REMOVAL OF PIGMENT-CONTAINING RESIDUES IN THE PHARMACEUTICAL OR COSMETICS INDUSTRY
ELIMINATION DE RESIDUS CONTENANT DES PIGMENTS DANS L'INDUSTRIE PHARMACEUTIQUE OU COSMETIQUE

(30) Priorität: 31.07.1999 DE 19936179
(43) Veröffentlichungstag der Anmeldung: 02.05.2002
(73) Patentinhaber: ECOLAB INC., St. Paul, MN 55102-1390 (US)
(72) Erfinder: BRAGULLA, Siegfried, D-40789 Monheim (DE); SERVE, Wilfried, D-51371 Leverkusen (DE)
(74) Vertreter: Sternagel, Fleischer, Godemeyer & Partner
(86) Internationale Anmeldenummer: PCT/EP2000/007051
(87) Internationale Veröffentlichungsnummer: WO 2001/009275

(56) Entgegenhaltungen:
- EP-A- 0 913 461
- DE-A- 4 310 995
- DE-A- 19 743 434
- PATENT ABSTRACTS OF JAPAN vol. 1999, no. 13, 30. November 1999 (1999-11-30) & JP 11 217592 A (NEOS CO LTD), 10. August 1999 (1999-08-10)

## Beschreibung

Die vorliegende Erfindung betrifft die Verwendung von Reinigungsmittelformulierungen in der pharmazeutischen und kosmetischen Industrie, zur Entfernung pigmenthaltiger Rückstände, die bei der Herstellung oder Abfüllung von pharmazeutischen oder kosmetischen Präparaten auftreten.

In der pharmazeutischen und kosmetischen Industrie verwendet man zur Reinigung von Oberflächen im Produktions- und Abfüllbereich üblicherweise hochalkalische und saure Reiniger im automatischen CIP-Verfahren (Cleaning in Place) oder im Umpump- oder Tauchverfahren.
Dabei werden im CIP-Verfahren üblicherweise Reinigungslösungen im Kreislauf gepumpt und beispielsweise zur effektiven Reinigung von Tanks über Spritzköpfe, die im Tank bereits installiert sind, versprüht.
Im Umpumpverfahren werden Reinigungslösungen mit einer Fließgeschwindigkeit von durchschnittlich 1,5 bis 4 m/sec in geschlossenen Kreisläufen umgepumpt.
Im Tauchverfahren werden kleine, demontierte Anlagenteile oder sonstige Gegenstände in Behältern, wie beispielsweise Wannen, in Reinigungslösungen getaucht. Nach dem Einweichen der Rückstände werden die gelösten Bestandteile mit klarem Wasser abgespült.

Zur Steigerung der Reinigungsleistung, insbesondere, wenn stark fetthaltige Verbindungen aus den Produktionsanlagen entfernt werden müssen, verwendet man stark tensidhaltige Reiniger. Die tensidhaltigen Reiniger haben dabei die Aufgabe, die Fettrückstände von den Oberflächen der Produktionsanlagen zu lösen, in der wäßrigen Reinigungslösung stabil zu emulgieren und somit aus den Produktionsanlagen auszuspülen.

Im Falle der Entfernung von Rückständen, die bei der Herstellung pigmenthaltiger Produkte (wie sie in kosmetischen und pharmazeutischen Produkten verwendet werden) auftreten, reicht die Reinigungskraft üblicher Tensidmischungen nicht aus.

Die in den pigmenthaltigen Produkten der pharmazeutischen und kosmetischen Industrie enthaltenen Pigmente bestehen aus organischen und anorganischen Substanzen, wie z.B. Eisenoxid, Titandioxid, Zinkoxid, organische Farbstoffe. Verwendung finden diese Pigmente in Produkten wie Sonnenschutzcrernes, Babycremes, Wimperntuschen sowie für Film- oder Lacktabletten oder Dragees.

Sehr viele dieser Cremes und Lotionen enthalten als weitere Komponente unterschiedliche Öle und Fette, wie z.B. Paraffinöl, Silikonöl, Vasseline, Wollwachs oder native Öle im Gemisch mit Emulgatoren und demineralisiertem Wasser.

Die in den pharmazeutischen und kosmetischen Präparaten vorliegenden Kombinationen von Pigmenten und Fetten und Ölen führt im Produktions- und Abfüllbereich zu großen Rückstandsproblemen. Die Pigmente bleiben nach gründlicher, chemischer Reinigung mit üblichen, alkalischen bzw. sauren, tensidhaitigen Reinigern auf den zu reinigenden Oberflächen als dünner Film zurück. Dieser Film läßt sich nach der chemischen Reinigung nur durch manuelles Abwischen entfernen.

Dies hat Nachteile wie personal-, zeit- und damit kostenintensive Reinigungsaktionen zur Folge, da nach jeder Produktionscharge aus hygienischen Gründen die gesamte Prozeßlinie gründlich gereinigt werden sollte, um mikrobiologisches Wachstum zu vermeiden. Besonders beim Produktwechsel auf einer Produktionsanlage müssen alle Rückstände des vorherigen Produktionsablaufes gründlich durch chemische Reinigung entfernt werden, um eine Kontamination der nachfolgenden Produktionscharge mit Resten der Vorproduktion zu vermeiden.

Aus der US-A-4534881 ist die Verwendung von Polyaspartat, Polyglutamat und Copolykondensaten mit anderen Aminosäuren zur Inhibierung von anorganischen oder biologischen Calciumcarbonatausfällungen bekannt. Die Polyaminosäuren werden beispielsweise in Meerwasseraufbereitungsanlagen oder Kühlkreisläufen als Gelinibita tubitor verwendet.

Gemäß der Lehre der DE-A-3626672 entsteht durch thermische Polykondensation von Maleinsäureamoniumsalz bei 120 - 150 °C Polyasparaginsäure, die in der sauren Form Ablagerungen der Härtebildner des Wassers verhindert und Ablagerungen dieser Art wieder auflösen kann. Das Polyaspartat kann in Geschirrspülmaschinen das Mattwerden von Glas verhindern.

Aus der DE-A-225310 ist bekannt, dass mit langkettigen Alkylaminen modifizierte Polyasparaginsäuren als Tenside in Waschmitteln verwendet werden.

Aus der EP-A-0454126 und der EP-A-05111037 ist die Verwendung von Polyasparaginsäure als Builder in Waschmittelformulierungen bekannt. Gemäß der Lehre der WO-A-92/15535 werden Polyasparaginsäure mit Molmassen von 1.000 - 5.000 als Dispergiermittel verwendet.

Die DE-A-4310995 offenbart hochalkalische Reinigungsmittelformulierungen für Molkereien, die eine wässrige Lösung von Polyasparaginsäure, einem C10-Oxoalkoholethoxilat sowie Natriumgluconat aufweist. Die Reinigungsmittelformulierung dient insbesondere zur Entfernung von Ablagerungen aus Milchstein, der im wesentlichen aus Kalziumphosphat, Eiweiß und anderen Komponenten besteht.

Der Erfindung liegt die Aufgabe zugrunde, Reinigungsmittel für die pharmazeutische und kosmetische Industrie zur Verfügung zu stellen. Diese Reinigungsmittel sollten ein gutes Reinigungsvermögen bei der Entfernung pigmenthaltiger Rück-stände, die im Produktions- und Abfüllbereich der kosmetischen und pharmazeutischen Industrie anfallen, aufweisen.

Dementsprechend betrifft die vorliegenden Erfindung die Verwendung von Reinigungsmittelformulierungen, die als Komponenten mit komplexbildenden Eigenschaften Polyasparaginsäure und Gluconsäure und deren Salze enthalten, zur Entfernung von pigmenthaltigen Rückständen, ausgewählt aus Eisenoxid, Titanoxid und Zinkoxid, die bei der Herstellung oder Abfüllung von pharmazeutischen oder kosmetischen Präparaten auftreten. Darüber hinaus können ein oder mehrere weitere Komponenten mit komplexbildenden Eigenschaften ausgewählt aus den Gruppen der Aminocarbonsäuren, Polyaminosäuren und P-freien Carbonsäuren und deren Salzen in den Reinigungsmittelformulierungen enthalten sein, wobei die Komponenten mit komplexbildenden Eigenschaften vorzugsweise ausgewählt sind aus Nitrilotriessigsäure, Ethylendiamintetraessigsäure, Methylglycindiessigsäure, Zitronensäure, Dicarboxymethyl-L-glutaminsäure, Serindiessigsäure, Imidosuccinsäure, und der Gruppe der Polycarbonsäuren sowie jeweils deren Salzen.
Als Polycarbonsäuren kommen beispielsweise Polyacrylsäuren und Copolymere aus Maleinsäureanhydrid und Acrylsäure sowie die Natriumsalze dieser Polymersäuren in Betracht. Handelsübliche Produkte sind z. B. Sokalan® CP 5 und PA 30 von BASF, Alcosperse® 175 und 177 von Alco, LMW® 45 N und SPO2 ND von Norsohaas. Zu den geeigneten nativen Polymeren gehören beispielsweise oxidierte Stärke (z. B. DE 42 28 786) und Polyaminosäuren wie Polyglutaminsäure, z. B. der Firmen Cygnus, Bayer, Rohm & Haas, Rhône-Poulenc oder SRCHEM.

Die Reinigungsmittelformulierungen können zusätzlich als Komponenten mit kompexbildenden Eigenschaften eine oder mehrere Komponenten ausgewählt aus Nitrilotriessigsäure, oder eine Polycarbonsäure, die vorzugsweise auf Polymerisation von Asparaginsäure mit anderen Carbonsäuren zurückgeht enthalten.

Die erfindungsgemäß zu verwendenden Reinigungsmittelformulierungen enthalten zusätzlich nichtionisches Tensid, wobei das nichtionische Tensid vorzugsweise ausgewählt ist aus der Gruppe alkoxylierter Fettalkohole, die gegebenenfalls endgruppenverschlossen sind und / oder der Alkylpolyglycoside und /oder alkoxylierter Fettamine.

Als alkoxylierte Fettalkohole kommen beispielsweise C₈-C₁₈-Alkylpolyethylen-glykolpolypropylenglykolether mit jeweils bis zu 8 Mol Ethylenoxid(=EO)- und Propylenoxid(=PO)-Einheiten im Molekül, in Frage. Außerdem hat der Zusatz von mit 30 EO-Gruppen ethoxyliertem Talgalkohol sowie der Zusatz von mit 5 EO-Gruppen ethoxyliertem Oleyl-Cetylalkohol positiven Einfluß auf das Reinigungsergebnis gezeigt. Man kann aber auch andere bekannte nichtionische Tenside verwenden, wie z.B. C₁₂-C₁₈-Alkylpolyethylenglycolpolybutylenglykolether mit jeweils bis zu 8 Mol Ethylenoxid- und Butylenoxideinheiten im Molekül sowie endgruppenverschlossene Alkylpolyalkylenglykolmischether. Als alkoxylierte Fettamine kommen beispielsweise mit 8-16 EO-Gruppen ethoxylierte C₈-C₁₈-Alkylamine in Betracht.

Die erfindungsgemäß zu verwendenden Reinigungsmittelformulierungen enthalten in einer ebenfalls bevorzugten Ausführung zusätzlich Alkalitätsspender. Dabei kommen vorzugsweise Alkalihydroxide in Betracht. Der Anteil in den Reinigungmittelformulierungen liegt bevorzugt bei 2-50 Gew.% bezogen auf das Gesamtgewicht der Reinigungsmittelformulierungen. Die anwendungsfertige Reinigungslösung weist einen pH-Wert von 8 bis 14, vorzugsweise 10 bis 13 auf.

Bei der erfindungsgemäßen Verwendung werden die Komponenten mit komplexbildenden Eigenschaften und die nichtionischen Tenside gegebenenfalls zusammen mit weiteren Hilfsstoffen, wie Lösungsvermittlern, entweder
a) in einer einzigen vorzugsweise alkalischen Reinigungsmittelformulierung konfektioniert, die nach Verdünnen mit Wasser um einen Verdünnungsfaktor die Reinigungslösung ergibt, oder
b) separat in zwei verschiedenen Reinigungsmittelformulierungen untergebracht, die zur Herstellung der Reinigungslösung mit Wasser um einen gleichen oder zwei verschiedene Verdünnungsfaktoren verdünnt und zusammengebracht werden, oder zuerst zusammengebracht und dann um einen Verdünnungsfaktor mit Wasser verdünnt werden,
so daß in der anwendungsfertigen Reinigungslösung 0,025 bis 50 g/L, vorzugsweise 0,25 bis 10 g/L Komponenten mit komplexbildenden Eigenschaften und 0,05 bis 5 g/L, vorzugsweise 0,5 bis2 g/L nichtionisches Tensid, bezogen auf die gesamte Reinigungslösung, enthalten sind.

Der Anteil an Komponenten mit komplexbildenden Eigenschaften liegt bei der Verwendung von einer einzigen Formulierung vorzugsweise zwischen 1 und 75 Gew.-%, bezogen auf das Gesamtgewicht der Reinigungsmittelformulierung, während der Anteil an nichtionischen Tensiden bei der Verwendung von einer einzigen Formulierung vorzugsweise zwischen 2 und 50 Gew.-% bezogen auf das Gesamtgewicht der Reinigungsmittelformulierung liegt.
Bei Verwendung von zwei Formulierungen sind die entsprechenden Mengen an nichtionischen Tensiden und Komplexbildnern in getrennten Reinigungsmittelformulierungen enthalten. Die Formulierung mit dem nichtionischen Tensid ist vorzugsweise alkalisch eingestellt.

Gegenstand der Erfindung ist auch eine Reinigungsmittelformulierung auf Basis von Polyasparaginsäure, vorzugsweise in Mengen von 0,5 bis 30 Gew.-%, bezogen auf das Gesamtgewicht der Reinigungsmittelformulierung, und Gluconsäure, vorzugsweise in Mengen von 0,5 bis 45 Gew.-%, bezogen auf das Gesamtgewicht der Reinigungsmittelformulierung, die zusätzlich nichtionisches Tensid enthält, das vorzugsweise ausgewählt ist aus der Gruppe alkoxylierter Fettalkohole, die gegebenenfalls endgruppenverschlossen sind und/oder der Alkylpolyglycoside und /oder alkoxylierter Fettamine.

In einer bevorzugten Ausführung sind in der Reinigungsmittelformulierung ein oder mehrere Alkalitätsspender, vorzugsweise Alkalihydroxide, enthalten.

Die erfindungsgemäße Verwendung der beschriebenen Reinigungsmittelformulierungen in der pharmazeutischen und kosmetischen Industrie weist eine Reihe von Vorteilen auf.

So können durch die erfindungsgemäße Verwendung der beschriebenen Reinigungsmittelformulierungen die für die pharmazeutische und kosmetische Industrie spezifischen Verunreinigungenen, insbesondere pigmenthaltige mit Ölen und Fetten gemischten Rückstände, von Oberflächen entfernt werden.

Daraus ergeben sich Vorteile für den Produktions- und Abfüllprozess von kosmetischen und pharmazeutischen Produkten.
Es ist kein manuelles Nachwischen der Oberflächen zur Entfernung von pigmenthaltigen Rückständen erforderlich. Dies führt zur Reduzierung von Zeit-, Personal-und Kostenaufwand für die Reinigungszyklen.
Dadurch wird eine Prozessoptimierung im Produktions- und Abfüllbereich der pharmazeutischen und kosmetischen Industrie erreicht. Die Produktionszyklen können erhöht werden.

### Beispiele

Um die unterschiedliche Wirksamkeit von Reinigungsmittelformulierungen gegenüber Verschmutzungen, die für die pharmazeutische und kosmetische Industrie typisch sind, herauszuarbeiten, wurde folgender Test durchgeführt:
Edelstahlbleche (5 x 10 cm) wurden für den Test vorbereitet, indem auf einer Seite des Prüfbleches 0,4 bis 0,5 g von Standardschmutz aufgetragen wurde und man anschließend den Belag für 24 Stunden bei 25 °C trocknen ließ. Als Standardschmutz kamen zum einen ein Eisenoxid-haltiges Make up (Pigmentcreme 1) und zum anderen ein Titandioxid-haltiges Make up (Pigmentcreme 2) zum Einsatz.
Der Reinigungsversuch erfolgte durch Tauchen der so vorbereiteten Prüfobjekte in einer vollautomatischen Tauchapparatur in die verschiedenen Reinigungslösungen bei Temperaturen von 80 °C für 20 Minuten. Die Ermittlung der Belagsablösung wurde gravimetrisch durchgeführt.
Die in der folgenden Tabelle dargestellten Formulierungen wurden durch Verdünnen mit Wasser auf Anwendungskonzentration in eine Reinigungslösung überführt, deren Reinigungsleistung im Versuch ermittelt wurde.

**Tabelle 1:**

| Reinigungsleistung unterschiedlich zusammengesetzter Formulierungen gegenüber für die kosmetische Industrie üblichen Verunreinigungen | | | | | | | |
|---|---|---|---|---|---|---|---|
| | **Beispiele / Wirkstoffanteil in Gew%** | | | | | | |
| **Inhaltsstoffe** | **Vgl. 1** | **Vgl. 2** | **Vgl. 3** | **Vgl. 4** | **Vgl. 5** | **Bspl. 2** | **Bspl. 3** |
| NaOH | 25 | 20 | 25 | 50 | 25 | 25 | 50 |
| NTA | 0 | 0 | 0 | 0 | 4 | 0 | 0 |
| EDTA | 0 | 0 | 0 | 0 | 0 | 12 | 0 |
| Gluconsäure | 0 | 0 | 0 | 0 | 5 | 25 | 25 |
| Hydroxyethandiphos phonat (Na-Salz) | 2,5 | 2,5 | 2,5 | 2,5 | 2,5 | 2,5 | 2,5 |
| Polyasparaginsäure | 0 | 0 | 0 | 0 | | 5 | 20 |
| Fettalkohol C₁₂₋₁₈ 9 EO Butylether | | | | 16,5 | | | |
| Fettalkohol C₁₂₋₁₈ 5 EO +4 PO | 15 | 15 | 15 | | 15 | 15 | 15 |
| Kokosamin - 12 EO | 0 | 0 | 0 | 16,5 | 0 | 5 | 5 |
| Alkylglucosid | 2 | 1 | 2 | 0 | 5 | 0 | 0 |
| Rest auf 100 % | Demineralisiertes Wasser | | | | | | |
| AW-Konzentration (%) | 4 | 4 | 4 | 4 | 4 | 4 | 4 |
| Pigmententfernung | | | | | | | |
| Creme 1 | 11,3 | 1,6 | 11,8 | 16,4 | 78 | 98,5 | 98,7 |
| Creme 2 | 12,6 | 2,8 | 12,6 | 19,3 | 89 | 99,6 | 99,6 |
| Vgl. 1 bis 5 = Vergleichsbeispiele Bspl. 2 bis 3 = Erfindungsgemäße Beispiele | | | | | | | |

## Patentansprüche

1. Verwendung von Reinigungsmittelformulierungen, enthaltend eine Kombination aus Polyasparaginsäure und Gluconsäure oder deren Salzen als Komponenten mit komplexbildenden Eigenschaften sowie nichtionische Tenside, **dadurch gekennzeichnet, dass** sie zur Entfernung von pigmenthaltigen Rückständen, ausgewählt aus Eisenoxid, Titandioxid, und Zinkoxid , die bei der Herstellung oder Abfüllung von pharmazeutischen oder kosmetischen Präparaten auftreten, eingesetzt werden.

2. Verwendung nach Anspruch 1, **dadurch gekennzeichnet, dass** die Reinigungsmittelformulierungen zusätzlich weitere Komponenten mit komplexbildenden Eigenschaften enthalten.

3. Verwendung nach Anspruch 2, **dadurch gekennzeichnet, dass** als Komponenten mit komplexbildenden Eigenschaften Nitrilotriessigsäure, Ethylendiamintetraessigsäure, Methylglycindiessigsäure, Zitronensäure, Dicarboxymethyl-L-glutaminsäure, Serindiessigsäure, Imidosuccinsäure, Polycarbonsäuren, ausgewählt aus Polyacrylsäuren, Copolymeren aus Maleinsäureanhydrid und Acrylsäure, oxidierte Stärke, Polyglutaminsäure und Polycarbonsäuren, die auf Polymerisation von Asparaginsäure mit anderen Carbonsäuren basieren, sowie Hydroxiethandiphosphonsäure oder jeweils deren Salze eingesetzt werden.

4. Verwendung nach einem oder mehreren der Ansprüche 1 bis 3, **dadurch gekennzeichnet, dass** als nichtionische Tenside alkoxylierte Fettalkohole und/oder endgruppenverschlossene alkoxylierte Fettalkohole und/oder Alkylpolyglycoside und/oder alkoxylierte Fettamine eingesetzt werden.

5. Verwendung nach einem oder mehreren der Ansprüche 1 bis 4, **dadurch gekennzeichnet, dass** in den Reinigungsmittelformulierungen zusätzlich Alkalitätsspender enthalten sind.

6. Verwendung nach einem oder mehreren der Ansprüche 1 bis5, **dadurch gekennzeichnet, dass** die Komponenten mit komplexbildenden Eigenschaften und die nichtionischen Tenside gegebenenfalls zusammen mit weiteren Hilfsstoffen, wie Lösungsvermittlern entweder
a) in einer einzigen vorzugsweise alkalischen Reinigungsmittelformulierung enthalten sind, die nach Verdünnen mit Wasser um einen Verdünnungsfaktor die Reinigungslösung ergibt, oder
b) separat in zwei verschiedenen Reinigungsformulierungen untergebracht sind, die zur Herstellung der Reinigungslösung mit Wasser um einen gleichen oder zwei verschiedene Verdünnungsfaktoren verdünnt und zusammengebracht werden, oder zuerst zusammengebracht und dann um einen Verdünnungsfaktor mit Wasser verdünnt werden,
so dass in der anwendungsfertigen Reinigungslösung 0,025 bis 50 g/L Komponenten mit komplexbildenden Eigenschaften und 0,05 bis 5 g/L nichtionisches Tensid, bezogen auf die gesamte Reinigungslösung, enthalten sind.

## Claims

1. Use of cleaning agent formulations containing a combination of polyaspartic acid and gluconic acid or salts thereof as components with complexing properties, as well as non-ionic surfactants, **characterized in that** said formulations are employed for the removal of pigment-containing residues selected from iron oxide, titanium dioxide and zinc oxide which arise during production or packaging of pharmaceutical or cosmetic preparations.

2. The use according to claim 1, **characterized in that** the cleaning agent formulations additionally contain further components having complexing properties.

3. The use according to claim 2, **characterized in that** nitrilotriacetic acid, ethylenediaminetetraacetic acid, methylglycinediacetic acid, citric acid, dicarboxymethyl-L-glutamic acid, serinediacetic acid, imidosuccinic acid, polycarboxylic acids selected from polyacrylic acids, copolymers of maleic anhydride and acrylic acid, oxidized starch, polyglutamic acid and polycarboxylic acids based on polymerization of aspartic acid with various carboxylic acids, as well as hydroxyethanediphosphonic acid or the respective salts thereof are employed as components having complexing properties.

4. The use according to one or more of claims 1 to 3, **characterized in that** alkoxylated fatty alcohols and/or capped alkoxylated fatty alcohols and/or alkylpolyglycosides and/or alkoxylated fatty amines are employed as non-ionic surfactants.

5. The use according to one or more of claims 1 to 4, **characterized in that** the cleaning agent formulations additionally contain alkalinity sources.

6. The use according to one or more of claims 1 to 5, **characterized in that** the components with complexing properties and the non-ionic surfactants, optionally together with further auxiliary agents such as solubilizers, are contained either
a) in one single, preferably alkaline cleaning agent formulation which, following dilution with water by a dilution factor, furnishes the cleaning solution, or
b) separately in two different cleaning formulations which, in order to produce the cleaning solution, are diluted with water, using one identical or two different dilution factors, and combined, or combined first and subsequently diluted with water by a dilution factor,
so that the ready-to-use cleaning solution contains from 0.025 to 50 g/l of components with complexing properties and from 0.05 to 5 g/l of non-ionic surfactant, relative to the overall cleaning solution.

## Revendications

1. Utilisation de formulations de produits de nettoyage contenant une combinaison d'acide polyaspartique et d'acide gluconique ou de leurs sels comme composants présentant des propriétés de formation de complexes ainsi que des agents tensioactifs,
**caractérisée en ce qu'**
on les emploie pour éliminer des résidus contenant des pigments et choisis parmi l'oxyde de fer, le dioxyde de titane et l'oxyde de zinc, qui apparaissent lors de la fabrication ou du remplissage de préparations pharmaceutiques ou cosmétiques.

2. Utilisation selon la revendication 1,
**caractérisée en ce que**
les formulations de produits de nettoyage contiennent en outre d'autres composants présentant des propriétés de formation de complexes.

3. Utilisation selon la revendication 2,
**caractérisée en ce que**
comme composants présentant des propriétés de formation de complexes, on utilise l'acide nitrilotriacétique, l'acide éthylènediaminetétraacétique, l'acide méthylglycinediacétique, l'acide citrique, l'acide dicarboxyméthyl-L-glutamique, l'acide sérine-diacétique, l'acide imidosuccinique, des acides polycarboxyliques choisis parmi les acides polyacryliques, les copolymères de l'anhydride maléique et de l'acide acrylique, l'amidon oxydé, l'acide polyglutamique et les acides polycarboxyliques qui reposent sur la polymérisation de l'acide aspartique avec d'autres acides carboxyliques, ainsi que l'acide hydroxyéthanediphosphonique, ou respectivement leurs sels.

4. Utilisation selon une ou plusieurs des revendications 1 à 3,
**caractérisée en ce que**
comme agents tensioactifs non ioniques on utilise des alcools gras alcoxylés et/ou des alcools gras alcoxylés bloqués à leurs groupes d'extrémité et/ou des alkylpolyglycosides et/ou des amines grasses alcoxylées.

5. Utilisation selon une ou plusieurs des revendications 1 à 4,
**caractérisée en ce que**
dans les formulations de produits de nettoyage on trouve en outre des générateurs d'alcalinité.

6. Utilisation selon une ou plusieurs des revendications 1 à 5,
**caractérisée en ce que**
les composants présentant des propriétés de formation de complexes et les agents tensioactifs non ioniques sont, le cas échéant avec d'autres adjuvants, comme des tiers solvants,
a) soit contenus dans une seule formulation de produit de nettoyage de préférence alcaline, qui donne après dilution avec de l'eau selon un facteur de dilution la solution de nettoyage,
b) soit séparément mis dans deux formulations de nettoyage différentes, qui, pour produire la solution de nettoyage, sont diluées avec de l'eau d'un facteur de dilution identique ou de deux facteurs de dilution différents, et réunies, ou tout d'abord réunies puis diluées avec de l'eau d'un facteur de dilution donné,
de manière que dans la solution prête à l'emploi, on trouve de 0,025 à 50 g/l de composants présentant des propriétés de formation de complexes et de 0,05 à 5 g/l d'agent tensioactif non ionique, par rapport à l'ensemble de la solution de nettoyage.
